# EUROPEAN PATENT SPECIFICATION

(11) **EP 0 707 853 B1**
(45) Date of publication and mention of the grant of the patent: **30.07.1997**
(21) Application number: 95115576.1
(22) Date of filing: 02.10.1995
(51) Int. Cl.: A61K 31/58, A61K 9/08, A61K 47/18, A61K 47/24

(54) **Muscle relaxant pharmaceutical compositions**
Glattmuskelrelaxierende Arzneizubereitungen
Compositions pharmaceutiques à activité relaxante des muscles

(30) Priority: 21.10.1994 IT MI942166
(43) Date of publication of application: 24.04.1996
(73) Proprietor: POLI INDUSTRIA CHIMICA S.p.A., 20141 Milano (IT)
(72) Inventor: Poli, Stefano, I-20123 Milano (IT); Crimella, Tiziano, I-20154 Milano (IT); Magni, Ambrogio, I-22058 Osnago (Como) (IT); Moro, Luigi, I-21050 Cairate (Varese) (IT)
(74) Representative: Minoja, Fabrizio

(56) References cited:
- WO-A-93/15736
- WO-A-94/17808

## Description

Vecuronium bromide is a known neuromuscular blocking agent, employed in therapy as coadjuvant in surgical anaesthesia to obtain relaxation of skeletal muscles.

Normally, therapy is performed by i.v. administration of a suitable dosage form. This dosage form may be administered dissolving a freeze-dried powder, containing the active ingredient associated with some excipients by injection, in water or a suitable solvent. The excipients can be stabilizers.

The formulation of vecuronium bromide, presently utilized worldwide under the trademark NORCURON^{R}, is described in EP Patent 0 008 824 (Akzo Chemical) and is characterized by the presence of sodium phosphate and citric acid as stabiliser. The active ingredient in this composition is therefore in the form of an acid addition salt formed by reaction at the nonquaternarized nitrogen of the molecule.

The above-mentioned EP patent specification describes the use of acid addition salts to a monoquaternarized compound in position 16 of the 2,16-dipiperidine androstane nucleus. The formation of an addition salt is claimed to be a means to provide a product having the proper solubility and stability in solution, without which clinical use would be impossible.

Among the possible pharmaceutically acceptable addition salts made with organic or inorganic acids, the hydrochloride, bromhydrate, maleate, nitrate and phosphate salts are mentioned in EP Patent 0 008 824.

To prove that formation of the addition salts has a stabilising effect, chromatographic data showing a substantial degradation of an aqueous vecuronium bromide solution within few hours are compared to the results for the hydrochloride addition salt under the same conditions.

Since a solution of the sole active ingredient in water produces a basic reaction, the stability of vecuronium bromide solutions depends on the presence of acids.

WO 94/17808 (Inpharm N.V.) discloses a process for the preparation of vecuronium bromide aqueous solutions comprising the dissolution of the freeze-dried active principle in an amino-acid solution so as to obtain a final physiological pH (7.0-7.4). Freeze-drying is carried out in water saturated with CO₂ or in an organic solvent.

### Summary of the invention

It has been surprisingly found that stabilisation of vecuronium bromide or other steroidal muscle relaxant agents, such as pipecuronium bromide or rocuronium bromide, in aqueous solution can be obtained even without resorting to the production of addition salts of pharmaceutically acceptable organic or inorganic acids.

It is an object of the present invention to provide a stabilized vecuronium bromide solution or a stabilized solution of another muscle relaxant agent which is not based on making a pharmaceutically acceptable organic or inorganic acid addition salt.

According to the invention an efficacious solubilization of the active ingredient of a muscle relaxant composition, particularly vecuronium bromide, can, in fact, be obtained by including zwitterionic substances in the composition, even those classified as internal salts.

Examples of suitable zwitterionic substances for the composition according to the invention include amino acids with an isoionic point lower than 7, for example glycine, serine, methionine, alanine, isoleucine, leucine, phenylalanine, proline, hydroxyproline, tryptophan, tyrosine, valine and cysteine. Glycine and serine are particularly preferred.

Such substances cannot form addition salts with vecuronium bromide due to the dissociation constants of their ionic groups. It is known in fact from literature (AHFS American Hospital Formulatory Service, published by Amer. Soc. Hosp. Pharm., ed. 1992, page 736) that vecuronium bromide has a pKa of 8.97, corresponding to a pKb of 5.03. In the table below, pKa values for basic groups are listed jointly with corresponding values of their isoionic point for zwitterionic substances which can be used in the present invention.

**Table**

| Zwitterionic Substances for the Composition of the Invention | | |
|---|---|---|
| Zwitterionic substance | pKa | pI |
| alanine | 9,87 | 6,1 |
| glycine | 9,78 | 6,06 |
| hydroxyproline | 9,73 | 5,82 |
| serine | 9,15 | 5,68 |
| leucine | 9.24 | 6,04 |
| methionine | 10,6 | 5,91 |
| proline | 9,87 | 6,3 |
| valine | 9,72 | 6,0 |
| (data from "Handbook of Chemistry and Physics", C.R.C. Press, 1988-1989, ed. 69, page C 107). | | |

The pKa values of zwitterionic substances above are much greater than the corresponding value for vecuronium bromide. These substances are, in fact, stronger bases than vecuronium bromide and therefore, if kept at a pH equal to pI, the isoionic point, or, at any rate, greater than the pKb of vecuronium bromide, that means until pH higher than 5.1, they are able to preferentially form ion pairs with the ions originating from dissociation of acid groups present in the vecuronium bromide solution.

Experimental data, obtained by extraction with apolar organic solvent of freeze dried cakes prepared following the present invention, confirm that Vecuronium Bromide is quantitatively recovered by the solvent, so meaning that any vecuronium bromide salification due to acid addition does not take place in the composition. Applying the same experimental procedure on an acid addition salt of Vecuronium Bromide, prepared as detailed in EP 008.824, only a very modest level (about 20%) of active substance can be extracted by the solvent: that means that the main component of Norcuron^{R} is, as claimed, the acid addition salt of Vecuronium Bromide.

Maintaining the natural pH of solutions of vecuronium bromide containing the above zwitterionic substances is lower than 7, usually between 4.5 and 6.9 and preferably between 5 and 6, but solutions obtained with these substances are much more stable than the simple solutions in water of the compound.

The compositions of the invention turned out to be more stable than those disclosed in the above cited W0 94/17808, which differ also in the following aspects:
a) pH value;
b) concentration of amino-acids;
c) preparation method, comprising the freeze-drying of a carbon dioxide saturated water solution or organic solvent solution of vecuronium bromide.

Analytical control tests performed after preparation have in fact shown how some pharmaceutical compositions, prepared as explained in the present invention, show the stability profile typical to this class of compositions and, more generally, to the freeze-dried pharmaceutical injectable compositions. These composition must be stable for the few hours between preparation and administration. It must be noted that this new technological approach provides solutions having more physiological pH values and, by consequence, an enhanced tolerability pattern.

According to a further aspect of the invention, as zwitterionic substances able to stabilize neuromuscular blocking agents, like vecuronium, pipecuronium or rocuronium, phospholipids can also be used, more particularly, phosphatidylcholine and phosphatidylethanolamine. A monoprotic phosphoric group and an electron donor group which can form an internal salt coexist in these molecules. Due to their amphipatic nature, moreover, these substances are able to act as surfactants or cosurfactants in the formation of stable emulsions and microemulsions. Also under certain particular conditions, like the presence of excess water and absence of an oily phase, they can form the vesicular onion-like structures known as liposomes, able to entrap and mask the active ingredients and, consequently, solubilizing and stabilising them.

The pharmaceutical compositions of the present invention can be prepared by lyophilization of a drug solution containing in addition the suitable cryoprotectant as lyophilization support. The substances required to stabilize the solutions of neuromuscular blocking agents according to the invention, like vecuronium bromide, i.e. the zwitterionic compounds, are added to the solution to be freeze-dried in percentages ranging between 0.01% and 30%, preferably between 2% and 30%, and more preferably between 2 and 20% and even more preferably between 5 and 10% of the total weight of the injectable aqueous composition.

Generally the pharmaceutical compositions according to the invention contain an effective amount of the neuromuscular blocking agent. The preferred compositions of vecuronium bromide contain from 0.01 to 2.5% by weight, advantageously from 0.1 to 1% by weight of the active ingredient.

The following examples describe the invention in more details.

### EXAMPLES

### Example 1

In one liter of water suitable for an injectable preparation 1.8 g of methyl p-hydroxybenzoate and 0.2 g of propyl p-hydroxybenzoate are dissolved while heating. After cooling the solution, 105 g of glycine and, finally, 4.2 g of vecuronium bromide are added. The solution is filtered through a sterilizing membrane and divided into 1 ml ampoules which are frozen and subjected to lyophilization before sealing.

At the moment of use the solution is prepared using water for injectable preparations. The prepared product, kept for 24 hours at 4°C shows a slight decrease in potency, but not sufficient, at any rate, to jeopardize a positive conclusion regarding its conformity to the specifications for its activity.

### Example 2

A solution of 5% w/v glycine solution in water for injectable preparations is prepared in a 3 liter reactor. Vecuronium bromide is dissolved in this solution up to a 0.4% w/v concentration and the solution is brought, if necessary, to a pH equal to the isoionic point of glycine (6.1), after sterilizing filtration, before being divided into the ampoules, up to the unitary extractable volume of 2 ml. The ampoules are then frozen at -40°C and lyophilized up to a content in residue humidity of < 1%. Under these conditions ampoules prepared with water for injectable preparations remain unaltered, if preserved at the temperature of 4°C and show the presence of degradation products in analytically negligible quantities, if kept at room temperature for the 24 hours subsequent to preparation.

### Example 3

A 5% w/v serine solution is prepared in a 3 liter reactor in water for injectable preparations. Vecuronium bromide is dissolved up to a 0,4% w/v concentration in this solution and the solution is, if required, brought to a pH value equal to pI of serine (5.67) by addition of diluted HCl, after sterilising filtration, before being divided into ampoules. The ampoules are then frozen at -40°C and lyophilized up to a content of < 1% in residual moisture. Under these conditions ampoules prepared with water for injectable preparations remain unaltered if preserved at the temperature of 4°C (a HPLC analysis revealed a potency decrease ≤ 1% after 24 hours) and show the presence of degradation products in analytically negligible quantities, if kept at room temperature for the 24 hours subsequent to preparation.

### Example 4

Operating as described in Example 3 and with the same components ampoules are again prepared, bringing, after addition of vecuronium bromide, the pH of the solution again to a value of 5.2 by adding some drops of diluted inorganic acid. Stability profile of the preparation is similarly satisfying. After 3 days from the time of preparation, the ampoules, kept at +40°C do not show appreciable degradation products and the content of the active ingredient does not show any appreciable decrease.

### Example 5

A part of the ampoules containing the freeze-dried composition prepared as described in Example 3 are prepared using a suitable volume of an injectable micronised emulsion containing 20% of soja lipids, 1.2% of phospholipids and 2.25% of glycerol in water for injectables (this emulsion can be found already on the market, known under the registered trademark, Intralipid^{R}). The stability profile of the injectable emulsion of vecuronium bromide is similar to the one described for Example 3: after 4 days of storage at 4-10°C the potency decrease is not more than 3%.

### Example 6

In 30 g of warm ethyl alcohol 0.2 g of tocopherol acetate, 8 g of soja phosphatidylcholine and 1,5 g of cholesterol are dissolved and then 4.2 g of vecuronium bromide are added. 50 g of mannitol are dissolved separately in 800 ml of water and warmed to 60°C. The two solutions are mixed using a turboemulsifier homogeniser and brought to a final volume of one liter by addition of water for injectable solutions.

After filtration through an 0.45 micron membrane, the liposomic suspension is divided into vials and lyophilized according to the following procedure:
- freezing to -45°C for 5 hours on precooled plate;
- progressive desiccation to +40°C up to a final pressure value than 0,1 mbar;
- vaccum breakage with nitrogen and sealing the vials.

A liposomic suspension is prepared with a solution containing 0.1% glycine, 0.5% benzyl alcohol and a suitable amount of HCl to correct the pH to 5.5. The stability profile of the prepared suspension is satisfactory for at least 6 hours.

### Example 7

4 g of vecuronium bromide are mixed with 16 g of mannitol and subjected to sterilization through gamma ray irradiation. The sterile powder is then divided under aseptic conditions on a basis of 10 mg of active ingredient per vial and the vials are sealed with sterilised chlorobutyl plugs and protected by an aluminium cap. A 3.5% serine sterile solution is prepared separately also containing 2% benzyl alcohol. For the 24 hours subsequent to preparation of this solution, the active ingredient has a stability compatible with the use for which it is targeted.

### Example 8

A 10% w/v glycine solution in water in a 3 liter reactor for injectable preparations is used to dissolve vecuronium bromide up to a concentration of 0.4 % w/v. The pH is lowered up to 5.2 and the solution is filtered through a 0.22 micron sterilizing filter, taking care to receive the filtrate in a sterile room, where the solution is partitioned into glass type I vials to the individual volume of 2.5 ml in order to be lyophilized. A parenterally administrable solution can be obtained by preparing the freeze-dried powder with water for injectable or a 0.9% aqueous benzyl alcohol solution.

The stability of prepared solutions conforms to the requirements for administration that:
- in 0.9 % benzyl alcohol solution a potency decrease is not detected for as long as 5 days of storage in refrigerated conditions;
- in water for injection the potency is maintained for at least 24 hours in normal or refrigerated conditions.
Unless otherwise indicated % means percent by weight.

"Aqueous dispersion medium" means water and an additional solvent, for example ethanol or propylene glycol.

## Claims

1. Pharmaceutical composition for parenteral administration, said composition having a pH lower than 7 and comprising an effective amount of a steroidal neuromuscular blocking agent as active ingredient and from 0.01 to 30 % by weight of at least one zwitterionic substance having an isoionic point not higher than 7.

2. Pharmaceutical composition as defined in claim 1, wherein said steroidal neuromuscular blocking agent is vecuronium bromide in a concentration range from 0.01 to 2.5 % by weight.

3. Pharmaceutical composition as defined in claim 1 or 2, wherein the at least one zwitterionic substance is selected from the group consisting of glycine, serine, cysteine, valine, isoleucine, leucine, methionine, proline, hydroxyproline, alanine, phenylalanine, tyrosine and tryptophan.

4. Pharmaceutical composition as defined in any one of claims from 1 to 3, having a pH value of from 4.5 to 6.9.

5. Pharmaceutical composition as defined in claim 4, wherein said pH is from 5 to 6.

6. Pharmaceutical composition as defined in any one of claims from 1 to 5, wherein the at least one zwitterionic substance is glycine.

7. Pharmaceutical composition as defined in any one of claims from 1 to 5, wherein the at least one zwitterionic substance is serine.

8. Pharmaceutical composition as defined in any one of claims from 1 to 7, containing from 2 to 30 % by weight of the at least one zwitterionic substance.

9. Pharmaceutical composition as defined in any one of claims from 1 to 7, containing from 5 to 10 % by weight of the at least one zwitterionic substance.

10. Pharmaceutical composition as defined in any one of claims from 1 to 5, wherein the at least one zwitterionic substance is a phospholipid.

11. Pharmaceutical composition as defined in any one of claims from 1 to 10, further comprising phospholipidic vesicles containing at least one phospholipid and a prevailing aqueous dispersion medium.

12. Pharmaceutical composition as defined in any one of claims from 1 to 11, further comprising liposomes containing at least one phospholipid and a prevailing aqueous dispersion medium.

13. Pharmaceutical composition as defined in claim 12 containing a solvent phase consisting of a phospholipid-containing emulsion or microemulsion.

14. Pharmaceutical composition as defined in any one of claims from 1 to 13, further comprising a primary phospholipidic component selected from the group consisting of phosphatidylcholine, phosphatidylserine and phosphatidylethanolamine.

15. Pharmaceutical composition as defined in any one of claims from 1 to 14, in the form of a ready-to-use solution.

16. Pharmaceutical composition as defined in any one of claims from 1 to 15, in the form of a lyophilized solution and including a suitable solvent solution.

17. Method of making a pharmaceutical composition useful for administering a steroidal neuromuscular blocking agent comprising:
a) solubilizing the steroidal neuromuscular blocking agent and a zwitterionic stabilizer having an isoionic point not higher than 7, and lowering the final pH at a value near the isoionic point of this latter;
b) freeze-drying the solution obtained in a) to give a freeze-dried powdered composition containing an effective amount of the steroidal neuromuscular blocking agent to be reconstituted with sterile water, or other physiologically compatible solvent composition containing preservative substances, immediately before use.

18. Method of making a pharmaceutical composition as defined in claim 17, wherein said zwitterionic stabilizer is selected from the group consisting of glycine, serine, cysteine, valine, isoleucine, leucine, methionine, proline, hydroxyproline, alanine, phenylalanine, tyrosine, tryptophan, phosphatidylcholine, phosphatidylserine and phosphatidylethanolamine.

19. Method of making a pharmaceutical composition as defined in claim 17, wherein the final pH of the solution detailed under a) is at least 0.1 pH unit higher than the pKb value of the vecuronium bromide and, precisely, not lower than 5.2.

20. Pharmaceutical compositions obtainable by the methods of claims 17, 18 or 19.

## Patentansprüche

1. Pharmazeutisches Präparat zur parenteralen Verabreichung, wobei das Präparat einen pH-Wert von niedriger als 7 hat und eine wirksame Menge eines steroidischen neuromuskulären Blockierungsmittel als Wirkstoff und von 0.01 bis 30% Gew.-% mindenstens einer zwitterionischen Substanz, die einen isoionischen Punkt von nicht höher als 7 hat, enthält.

2. Pharmazeutisches Präparat nach Anspruch 1, dadurch gekennzeichnet, daß das steroidische neuromuskuläre Blockierungsmittel Vecuroniumbromid in einem Konzentrationsbereich von 0,01 bis 2,5 Gew.-% ist.

3. Pharmazeutisches Präparat nach Anspruch 1 oder 2 dadurch **gekennzeichnet**, daß die mindestens eine zwitterionische Substanz aus der Gruppe, bestehend aus Glycin, Serin, Cystein, Valin, Isoleucin, Leucin, Methionin, Prolin, Hydroxyprolin, Alanin, Phenylalanin, Tyrosin und Tryptophan ausgewählt ist.

4. Pharmazeutisches Präparat nach einem der Ansprüche 1 bis 3, dadurch **gekennzeichnet**, daß es einen pH-Wert von 4,5 bis 6,9 hat.

5. Pharmazeutisches Präparat nach Anspruch 4, dadurch **gekennzeichnet**, daß es einen pH-Wert von 5 bis 6 hat.

6. Pharmazeutisches Präparat nach einem der Ansprüche 1 bis 5, dadurch **gekennzeichnet**, daß die mindestens eine zwitterionische Substanz Glycin ist.

7. Pharmazeutisches Präparat nach einem der Ansprüche 1 bis 5, dadurch **gekennzeichnet**, daß die mindestens eine zwitterionische Substanz Serin ist.

8. Pharmazeutisches Präparat nach einem der Ansprüche 1 bis 7, dadurch **gekennzeichnet**, daß es 2 bis 30 Gew.-% der mindestens einen zwitterionischen Substanz enthält.

9. Pharmazeutisches Präparat nach einem der Ansprüche 1 bis 7, dadurch **gekennzeichnet**, daß es 5 bis 10 Gew.-% der mindestens einen zwitterionischen Substanz enthält.

10. Pharmazeutisches Präparat nach einem der Ansprüche 1 bis 5, dadurch **gekennzeichnet**, daß die mindestens eine zwitterionische Substanz ein Pospholipid ist.

11. Pharmazeutisches Präparat nach einem der Ansprüche 1 bis 10, dadurch **gekennzeichnet**, daß es weiterhin Phospholipidvesikel mit mindestens einem Phospholipid und ein vorwiegend wäßriges Dispersionsmedium enthält.

12. Pharmazeutisches Präparat nach einem der Ansprüche 1 bis 11, dadurch **gekennzeichnet**, daß es weiterhin Liposomen mit mindestens einem Phospholipid und ein vorwiegend wäßriges Dispersionsmedium enthält.

13. Pharmazeutisches Präparat nach Anspruch 12, dadurch **gekennzeichnet**, daß es eine Lösungsmittelphase enthält, die eine Phospholipid-enthaltende Emulsion oder Mikroemulsion enthält.

14. Pharmazeutisches Präparat nach einem der Ansprüche 1 bis 13, dadurch **gekennzeichnet**, daß es weiterhin einen primären Phospholipidbestandteil, ausgewählt aus der Gruppe, bestehend aus Phosphatidylcholin, Phosphatidylserin und Phosphatidylethanolamin, enthält.

15. Pharmazeutisches Präparat nach einem der Ansprüche 1 bis 14, dadurch **gekennzeichnet**, daß es in Form einer gebrauchsfertigen Lösung vorliegt.

16. Pharmazeutisches Präparat nach einem der Ansprüche 1 bis 15, dadurch **gekennzeichnet**, daß es in Form einer lyophilisierten Lösung und einschließlich eines geeigneten Lösungsmittels vorliegt.

17. Verfahren zur Herstellung eines pharmazeutischen Präparats, das zur Verabreichung eines steroidischen neuromuskulären Blockierungsmittel nützlich ist, dadurch gekennzeichnet daß
a) das steroidische neuromuskuläre Blockierungsmittel und ein zwitterionisches Stabilisierungsmittel mit einem isoionischen Punkt von nicht höher als 7 solubilisiert werden und der End-pH-Wert auf einen Wert nahe dem isoionischen Punkt des zwitterionischen Stabilisierungsmittels erniedrigt wird, und
b) die unter a) erhaltene Lösung gefriergetrocknet wird, um ein gefriergetrocknetes pulverisiertes Präparat zu erhalten, das eine wirksame Menge des steroidischen neuromuskulären Blockierungsmittel enthält und direkt vor der Verwendung in sterilem Wasser oder einer anderen physiologisch vertraglichen Lösungsmittelzusammensetzung, die konservierende Stoffe enthält, rekonstituiert wird.

18. Verfahren zur Herstellung eines pharmazeutischen Präparates nach Anspruch 17, dadurch **gekennzeichnet**, daß das zwitterionische Stabilisierungsmittel aus der Gruppe, bestehend aus Glycin, Serin, Cystein, Valin, Isoleucin, Leucin, Methionin, Prolin, Hydroxyprolin, Alanin, Phenylalanin, Tyrosin, Tryptophan, Phosphatidylcholin, Phosphatidylserin und Phosphatidylethanolamin ausgewählt wird.

19. Verfahren zur Herstellung eines pharmazeutischen Präparates nach Anspruch 17, dadurch **gekennzeichnet**, daß der pH-Wert der nach a) erhaltenen Lösung, mindestens 0,1 pH-Einheiten über dem pKb-Wert des Vecuroniumbromides und exakt nicht unter 5,2 ist.

20. Pharmazeutische Präparate erhältlich nach den Verfahren der Ansprüche 17, 18 oder 19.

## Revendications

1. Composition pharmaceutique pour administration parentérale, ladite composition ayant un pH inférieur à 7, et comprenant une quantité efficace d'un agent bloquant neuromusculaire stéroïdal comme ingrédient actif et de 0,01 a 30% en poids d'au moins une substance zwitterionique ayant un point iso-ionique non supérieur à 7.

2. Composition pharmaceutique telle que définie dans la revendication 1, dans laquelle ledit agent bloquant neuromusculaire stéroidal est du bromure du vécuronium a une concentration comprise entre 0,01 et 2,5 % en poids.

3. Composition pharmaceutique telle que définie dans la revendication 1 ou la revendication 2, dans laquelle ladite au moins une substance zwitterionique est choisie dans l'ensemble constitué de la glycine, la sérine, la cystéine, la valine, l'isoleucine, la leucine, la méthionine, la proline, l'hydroxyproline, l'alanine, la phénylalanine, la tyrosine, et le tryptophane.

4. Composition pharmaceutique telle que définie dans l'une quelconque des revendications 1 à 3, dont la valeur du pH est comprise entre 4,5 et 6,9.

5. Composition pharmaceutique telle que définie dans la revendication 4, dans laquelle le pH est compris entre 5 et 6.

6. Composition pharmaceutique telle que définie dans l'une quelconque des revendications 1 à 5, dans laquelle ladite au moins une substance zwitterionique est la glycine.

7. Composition pharmaceutique telle que définie dans l'une quelconque des revendications 1 à 5, dans laquelle ladite au moins une substance zwitterionique est la sérine.

8. Composition pharmaceutique telle que définie dans l'une quelconque des revendications 1 à 7, contenant de 2 à 30 % en poids de ladite au moins une substance zwitterionique.

9. Composition pharmaceutique telle que définie dans l'une quelconque des revendications 1 à 7, contenant de 5 à 10 % en poids de ladite au moins une substance zwitterionique.

10. Composition pharmaceutique telle que définie dans l'une quelconque des revendications 1 à 5, dans laquelle ladite au moins une substance zwitterionique est un phospholipide.

11. Composition pharmaceutique telle que définie dans l'une quelconque des revendications 1 à 10, comprenant en outre des vésicules phospholipidiques contenant au moins un phospholipide et un milieu de dispersion aqueux majoritaire.

12. Composition pharmaceutique telle que définie dans l'une quelconque des revendications 1 à 11, comprenant en outre des liposomes contenant au moins un phospholipide et un milieu de dispersion aqueux majoritaire.

13. Composition pharmaceutique telle que définie dans la revendication 12, contenant une phase de solvant constituée d'une émulsion ou d'une microémulsion contenant des phospholipides.

14. Composition pharmaceutique telle que définie dans l'une quelconque des revendications 1 à 13, comprenant en outre un composant phospholipidique primaire choisi dans l'ensemble constitué de la phosphatidylcholine, de la phosphatidylsérine et de la phosphatidyléthanolamine.

15. Composition pharmaceutique telle que définie dans l'une quelconque des revendications 1 à 14, sous forme d'une solution prête à l'emploi.

16. Composition pharmaceutique telle que définie dans l'une quelconque des revendications 1 à 15, sous forme d'une solution lyophilisée et comprenant une solution disolvante appropriée.

17. Procédé de préparation d'une composition pharmaceutique utile pour l'administration d'un agent bloquant neuromusculaire stéroïdal comprenant les étapes consistant à:
a) solubiliser l'agent bloquant neuromusculaire stéroïdal et un stabilisant zwitterionique ayant un point isoionique non supérior à 7, et abaisser le pH final à une valeur proche du point iso-ionique de ce dernier;
b) lyophiliser la solution obtenue en a) pour obtenir une composition lyophilisée en poudre contenant une quantité efficace de l'agent bloquant neuromusculaire stéroidal, à reconstituer avec l'eau stérile, ou autre composition disolvante physiologiquement compatible contenant des substances conservatrices, immédiatement avant utilisation.

18. Procédé de préparation d'une composition pharmaceutique tel que défini dans la revendication 17, dans lequel ledit stabilisant zwitterionique est choisi dans le groupe constitué de la glycine, la sérine, la cystéine, la valine, l'isoleucine, la leucine, la méthionine, la proline, l'hydroxyproline, l'alanine, la phénylalanine, la tyrosine, le tryptophane, la phosphatidylcholine, la phosphatidylsérine et la phosphatidyléthanolamine.

19. Procédé de préparation d'une composition pharmaceutique tel que défini dans la revendication 17, dans lequel le pH final de la solution détaillée en a) est supérieur d'au moins 0,1 unité pH à la valeur du pKb du bromure de vécuronium, et n'est pas, précisément, inférieur à 5,2.

20. Compositions pharmaceutiques pouvant être obtenues par les procédés des revendications 17, 18 ou 19.
